# EUROPEAN PATENT APPLICATION

(11) **EP 3 769 806 A1**
(43) Date of publication of application: **27.01.2021**
(21) Application number: 18910744.4
(22) Date of filing: 23.03.2018
(51) Int. Cl.: A61M 25/10

(54) **BALLOON CATHETER**

(71) Applicant: ASAHI INTECC CO., LTD., Seto-shi, Aichi 489-0071 (JP)
(72) Inventor: MIKI Akihiro, Seto-shi, Aichi 489-0071 (JP); ERIKAWA Yutaka, Seto-shi, Aichi 489-0071 (JP)
(74) Representative: Winter, Brandl, Fürniss, Hübner, Röss, Kaiser, Polte - Partnerschaft mbB
(86) International application number: PCT/JP2018/011780
(87) International publication number: WO 2019/180928

(57) **Abstract**

The present invention is provided with: a balloon 5; an outer shaft 3 that is coupled to the proximal end of the balloon 5 with a coupling part j2; an inner shaft 9 that is inserted into the outer shaft 3 and that is coupled to the distal end of the balloon 5 with a coupling part j1; and a distal end-side marker 7a and a proximal end-side marker 7b disposed on the inner shaft 9, wherein both ends of the distal end-side marker 7a are disposed astride a proximal end coupling position j1p of the coupling part j1 for coupling the balloon 5 and the inner shaft 9, and both ends of the proximal end-side marker 7b are disposed astride a proximal end coupling position j2p of the coupling part j2 for coupling the balloon 5 and the outer shaft 3.

## Description

### TECHNICAL FIELD

The present invention relates to a balloon catheter used in the medical field.

### BACKGROUND ART

Conventionally, a therapeutic balloon catheter that restores the flow of blood or digestive fluid by expanding a stenosis formed inside a body lumen such as a blood vessel of a patient, and a fixing balloon catheter that makes a medical device such as a guide wire inserted therein to be more easily operated by expanding and fixing a balloon to a blood vessel wall or a digestive organ wall, are known.

A balloon catheter is typically provided with a balloon that can be expanded and contracted, an outer shaft that is coupled to the rear end of the balloon, and an inner shaft that is inserted into the balloon and the outer shaft and that is coupled to the front end of the balloon. Here, the inner shaft used for inserting a guide wire, and a lumen provided between the outer shaft and the inner shaft is used to charge and discharge a liquid (a liquid such as a physiological saline or a contrast agent) for expanding and contracting the balloon.

Furthermore, a radiopaque marker is provided inside the balloon of the balloon catheter so that the position of the balloon can be confirmed during a procedure.

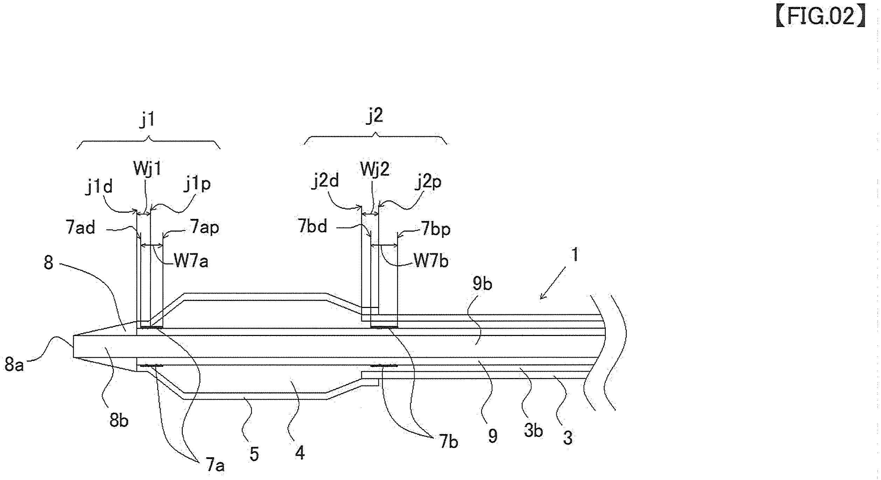

For example, Patent Document 1 describes a balloon catheter including a balloon, an outer tube shaft (hereinafter, referred to as an outer shaft) that is coupled to the proximal end of the balloon, an inner tube shaft (hereinafter, referred to as an inner shaft) that is inserted into the outer shaft and that is coupled to the distal end of the balloon, and a distal end-side contrast marker and a proximal end-side contrast marker which are formed on the inner shaft (see FIG. 2 and the like).

In the balloon catheter described in Patent Document 1, the distal end of the balloon that constitutes the balloon catheter is coupled to the inner shaft, and the proximal end of the balloon is coupled to the outer shaft.

However, when the balloon catheter is inserted into a body lumen of a patient and the balloon becomes curved, stress concentrates on the coupling part for coupling the balloon and the outer shaft and the coupling part for coupling the balloon and the inner shaft, which causes a problem in that peeling occurs more easily at the coupling parts.

Furthermore, in the balloon catheter described in Patent Document 1, because the proximal end-side contrast marker is disposed within a coupling width of the coupling part for coupling the balloon and the outer shaft, stress is further concentrated on the coupling part for coupling the balloon and the outer shaft, which causes a problem in that peeling occurs even more easily at the coupling parts.

### CITATION LIST

### Patent Document

Patent Document 1: Japanese Patent No. 6,103,721

### SUMMARY OF INVENTION

### Problems to be Solved by the Invention

The present invention has been made in response to the above problems associated the conventional techniques, and has an object of providing a balloon catheter that can prevent peeling between a balloon and an outer shaft or between the balloon and an inner shaft, and can prevent kinking near the end section of the balloon even when the balloon catheter is curved.

### Means for Solving the Problem

In order to solve the above problems, a first aspect of the present invention is a balloon catheter provided with a balloon, an outer shaft that is coupled to a proximal end of the balloon with a first coupling part, an inner shaft that is inserted into the outer shaft and that is coupled to a distal end of the balloon with a second coupling part, and a marker disposed on the inner shaft, wherein both ends of the marker are disposed astride at least one of, a distal end and a proximal end of the first coupling part, and a distal end and a proximal end of the second coupling part in a longitudinal direction of the inner shaft.

Furthermore, a second aspect of the present invention is the balloon catheter according to the first aspect, in which the both ends of the marker are disposed astride the distal end and the proximal end of the first coupling part, or the proximal end and the distal end of the second coupling part in the longitudinal direction of the inner shaft.

Moreover, a third aspect of the present invention is the balloon catheter according to the first aspect or the second aspect, in which the distal end of the outer shaft extends beyond the distal end of the first coupling part on a distal end side.

Also, a fourth aspect of the present invention is the balloon catheter according to the third aspect, in which a distal end of the marker extends beyond the distal end of the outer shaft on the distal end side.

In addition, a fifth aspect of the present invention is the balloon catheter according to any one of the first aspect to the fourth aspect, in which the marker is not disposed on the second coupling part, and is disposed on the first coupling part.

### Effects of the Invention

According to the first aspect of the present invention, a balloon catheter is provided with a balloon, an outer shaft that is coupled to a proximal end of the balloon with a first coupling part, an inner shaft that is inserted into the outer shaft and that is coupled to a distal end of the balloon with a second coupling part, and a marker disposed on the inner shaft, in which both ends of the marker are disposed astride at least one of, a distal end and a proximal end of the first coupling part, and a distal end and a proximal end of the second coupling part in a longitudinal direction of the inner shaft, and therefore, peeling can be prevented between the balloon and the outer shaft or between the balloon and the inner shaft, and kinking can be prevented near the end section of the balloon even when the balloon catheter is curved.

Furthermore, the second aspect of the present invention is the balloon catheter according to the first aspect, in which the both ends of the marker are disposed astride the distal end and the proximal end of the first coupling part, or the distal end and the proximal end of the second coupling part in a longitudinal direction of the inner shaft, and therefore, peeling can be further prevented between the balloon and the outer shaft or between the balloon and the inner shaft, and kinking can be further prevented near the end section of the balloon even when the balloon catheter is curved.

Moreover, the third aspect of the present invention is the balloon catheter according to the first aspect or the second aspect, in which the distal end of the outer shaft extends beyond the first coupling part on the distal end side, and therefore, in addition to the effects of the balloon catheter of the first aspect or the second aspect, the gradual change in rigidity in the longitudinal direction near the proximal end section of the balloon can be made smoother, and further, peeling can be further prevented between the balloon and the outer shaft, and kinking can be further prevented near the end section of the balloon even when the balloon catheter is curved.

Also, the fourth aspect of the present invention is the balloon catheter according to the third aspect, in which a distal end of the marker extends beyond the distal end of the outer shaft on the distal end side, and therefore, in addition to the effects of the balloon catheter of the third aspect, peeling can be further prevented between the balloon and the outer shaft, and kinking can be further prevented near the end section of the balloon even when the balloon catheter is curved, and further, the balloon can be deflated more safely by preventing the necking phenomenon from occurring at the proximal end section of the balloon.

In addition, the fifth aspect of the present invention is the balloon catheter according to any one of the first aspect to the fourth aspect, wherein the marker is not disposed on the second coupling part, and is disposed on the first coupling part, and therefore, in addition to the effects of the balloon catheter of any one of the first aspect to the fourth aspect, the distal end section of the balloon catheter can be made even thinner and softer.

### BRIEF DESCRIPTION OF DRAWINGS

FIG. 1 is an overall view of a balloon catheter according to a first embodiment of the present invention.
FIG. 2 is a longitudinal sectional view near the distal end section of the balloon catheter, which is an enlarged view of section A in FIG. 1.
FIG. 3 is a longitudinal sectional view near the distal end section of a balloon catheter according to a second embodiment.
FIG. 4 is a longitudinal sectional view near the distal end section of a balloon catheter according to a third embodiment.
FIG. 5 is a longitudinal sectional view near the distal end section of a balloon catheter according to a fourth embodiment.
FIG. 6 is a longitudinal sectional view near the distal end section of a balloon catheter according to a fifth embodiment.
FIG. 7 is a longitudinal sectional view near the distal end section of a balloon catheter according to a sixth embodiment.

### EMBODIMENTS OF THE INVENTION

Hereinafter, embodiments of the present invention will be described with reference to the drawings.

### (First Embodiment)

First, a first embodiment of the present invention will be described. The drawings used in the present embodiment are exaggerated for ease of understanding, and the dimensions are different from the actual dimensions.

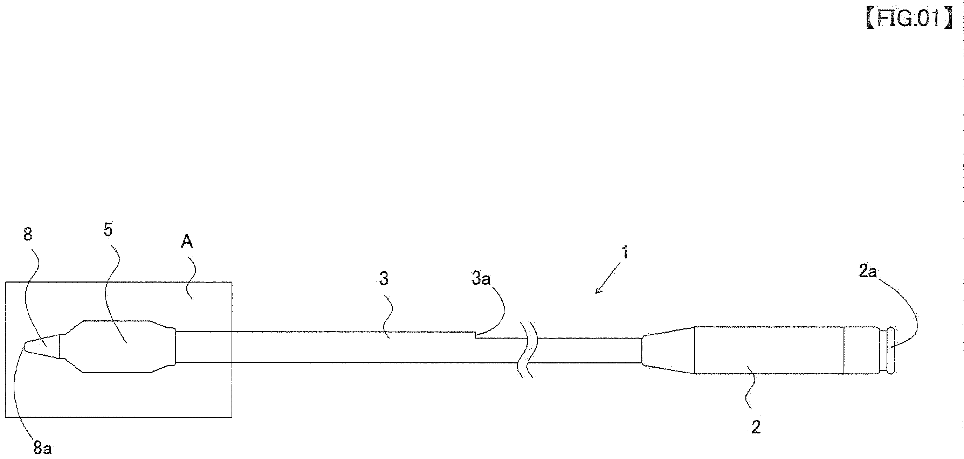

FIG. 1 is an overall view of a balloon catheter of the first embodiment of the present invention, and FIG. 2 is a longitudinal sectional view near the distal end section of the balloon catheter, which is an enlarged view of section A in FIG. 1.

As shown in FIG. 1, the balloon catheter 1 of the present embodiment includes a long outer shaft 3, a balloon 5 provided on the distal end of the outer shaft 3, a distal tip 8 provided on the distal end of the balloon 5, an inner shaft 9 that is inserted into the outer shaft 3 and the balloon 5 and that is coupled to the distal end of the balloon 5 (see FIG. 2), and a connector 2 provided on the proximal end of the outer shaft 3.

The outer shaft 3 is a long and flexible tubular member, and as shown in FIG. 2, the distal end is coupled to the proximal end of the balloon 5, and the proximal end is coupled to the distal end of the connector 2. The outer shaft 3 has an inner shaft 9 inserted therein, and has a lumen 3b between the outer shaft and the inner shaft 9 that communicates the internal space 4 of the balloon 5 with a lumen inside the connector 2 (not illustrated).

Furthermore, the outer shaft 3 is provided with a guide wire port 3a along the longitudinal direction, which serves as an opening for inserting a guide wire, and the guide wire port 3a is coupled to the proximal end of the inner shaft 9.

The material forming the outer shaft 3 is a flexible material, and examples include polyolefins, such as polyethylene, polypropylene, ethylene-propylene copolymer, and ethylene-vinyl acetate copolymer, thermoplastic resins such as soft polyvinyl chloride, various rubbers such as silicone rubber and latex rubber, various elastomers such as polyurethane elastomers, polyamide elastomers, and polyester elastomers, and crystalline plastics such as polyamide, crystalline polyethylene, and crystalline polypropylene, and further, any material having biocompatibility can be used without particular limitation.

The balloon 5 is configured to expand and contract in response to increases and decreases in the internal volume caused by changes in the pressure inside the balloon 5. Specifically, the balloon 5 is constituted by a body portion that expands into a cylindrical shape, a distal end-side cone part which is provided such that the outer diameter gradually decreases from the body portion toward the distal end, and a proximal end-side cone part which is provided such that the outer diameter gradually decreases from the body portion toward the proximal end, and an internal space 4 is formed inside the balloon 5 when it expands.

As shown in FIG. 2, the balloon 5 has the distal end coupled to the distal end of the inner shaft 9, and the proximal end coupled to the distal end of the outer shaft 3. Therefore, a liquid such as a physiological saline which is charged from the opening 2a of the connector 2 described below passes through the inside of a lumen (not illustrated) of the connector 2 and the lumen 3b, and is stored in the internal space 4 inside the balloon 5, and the balloon 5 expands and contracts according to the amount of stored liquid.

The material forming the balloon 5 is preferably a material having some softness, and examples include polyolefins such as polyethylene, polypropylene, and ethylene-propylene copolymer, polyesters such as polyethylene terephthalate, thermoplastic resins such as polyvinyl chloride, ethylene-vinyl acetate copolymer, crosslinked ethylene-vinyl acetate copolymer, and polyurethane, polyamide, polyamide elastomers, silicone rubber, and latex rubber.

Furthermore, the balloon 5 may be formed having a single layer structure or having a laminated structure with two or more layers using the above materials. Moreover, the surface of the balloon 5 may be coated with a substance having an antithrombotic property.

The distal tip 8 is connected to the distal end of the balloon 5 and the distal end of the inner shaft 9, is provided with a lumen 8b which communicates with the lumen 9b of the inner shaft 9, and an opening 8a that constitutes the distal end of the lumen 8b, and has a tapered shape in which the outer diameter gradually decreases toward the distal end.

The material forming the distal tip 8 is preferably a material having some flexibility, and examples include polyurethane elastomers, polyolefins such as polyethylene, polypropylene, ethylene-propylene copolymer, and ethylene-vinyl acetate copolymer, thermoplastic resins such as soft polyvinyl chloride, polyamide, polyamide elastomers, and polyurethane, silicone rubber, and latex rubber, and the thermoplastic resins above are particularly preferable.

The inner shaft 9 is a long and flexible tubular member which is inserted inside the outer shaft 3 and the balloon 5. The distal end of the inner shaft 9 is coupled to the distal end of the balloon 5, and the proximal end of the inner shaft 9 is coupled to the guide wire port 3a of the outer shaft 3 and is provided with a lumen 9b therein.

The same material as the material forming the outer shaft 3 described above can be used as the material forming the inner shaft 9. The material is a flexible material, and examples include polyolefins, such as polyethylene, polypropylene, ethylene-propylene copolymer, and ethylene-vinyl acetate copolymer, thermoplastic resins such as soft polyvinyl chloride, various rubbers such as silicone rubber and latex rubber, various elastomers such as polyurethane elastomers, polyamide elastomers, and polyester elastomers, and crystalline plastics such as polyamide, crystalline polyethylene, and crystalline polypropylene, and any material having biocompatibility can be used without particular limitation.

Furthermore, the inner shaft 9 of the present embodiment is provided with two radiopaque markers, namely a distal end-side marker 7a and a proximal end-side marker 7b which enable the position of the balloon 5 to be confirmed during a procedure.

Next, the relationship between the coupling position between the balloon 5 and the inner shaft 9, and the position of the distal end-side marker 7a will be described.

First, as shown in FIG. 2, the coupling position of the coupling part j1 between the balloon 5 and the inner shaft 9 (which corresponds to a "second coupling part" of the present invention) has a distal end coupling position j1d at the distal end, and a proximal end coupling position j1p at the position of a coupling width Wj1 on the proximal end side from the distal end coupling position j1d.

In contrast, the distal end position 7ad of the distal end-side marker 7a is positioned on the proximal end side of the distal end coupling position j1d of the coupling part j1 in the longitudinal direction of the inner shaft 9 (or the balloon catheter 1), and the proximal end position 7ap is at the position of a width W7a on the proximal end side from the distal end position 7ad, and is positioned on the proximal end side of the proximal end coupling position j1p of the coupling part j 1 in the longitudinal direction of the inner shaft 9 (or the balloon catheter 1).

That is to say, both ends of the distal end-side marker 7a are disposed astride the proximal end coupling position j1p of the coupling part j1 between the balloon 5 and the inner shaft 9 in the longitudinal direction of the inner shaft 9 (or the balloon catheter 1).

Therefore, because the both ends of the distal end-side marker 7a are disposed astride the proximal end coupling position j1p of the coupling part j1 between the balloon 5 and the inner shaft 9 in the longitudinal direction of the inner shaft 9 (or the balloon catheter 1), peeling can be prevented at the proximal end coupling position j1p between the balloon 5 and the inner shaft 9, and kinking can be prevented near the end section of the balloon 5 even when the balloon catheter 1 is curved.

Next, the relationship between the coupling position between the balloon 5 and the outer shaft 3, and the position of the proximal end-side marker 7b will be described.

As shown in FIG. 2, the coupling position of the coupling part j2 between the balloon 5 and the outer shaft 3 (which corresponds to a "first coupling part" of the present invention) has a distal end coupling position j2d at the distal end, and a proximal end coupling position j2p at the position of a coupling width Wj2 on the proximal end side from the distal end coupling position j2d.

In contrast, the distal end position 7bd of the proximal end-side marker 7b is positioned on the proximal end side of the distal end coupling position j2d of the coupling part j2 in the longitudinal direction of the inner shaft 9 (or the balloon catheter 1), and the proximal end position 7bp is at the position of a width W7b on the proximal end side from the distal end position 7bd, and is positioned on the proximal end side of the proximal end coupling position j2p of the coupling part j2 in the longitudinal direction of the inner shaft 9 (or the balloon catheter 1).

That is to say, both ends of the proximal end-side marker 7b are disposed astride the proximal end coupling position j2p of the coupling part j2 between the balloon 5 and the outer shaft 3 in the longitudinal direction of the inner shaft 9 (or the balloon catheter 1).

Therefore, because the both ends of the proximal end-side marker 7b are disposed astride the proximal end coupling position j2p of the coupling part j2 between the balloon 5 and the outer shaft 3 in the longitudinal direction of the inner shaft 9 (or the balloon catheter 1), peeling can be prevented at the proximal end coupling position j2p between the balloon 5 and the outer shaft 3, and kinking can be prevented near the end section of the balloon 5 even when the balloon catheter 1 is curved.

Examples of the material that can be used to form the distal end-side marker 7a and the proximal end-side marker 7b include radiopaque metals or metallic compounds such as tungsten, gold, platinum, indium, platinum-iridium alloy, and tantalum, and radiopaque resins formed by kneading a radiopaque material such as tungsten, bismuth trioxide, or barium sulfate with a resin material.

The connector 2 is coupled to the proximal end of the outer shaft 3, includes a lumen (not illustrated) that communicates with the lumen 3b of the outer shaft 3, and an opening 2a on the proximal end of the lumen, such that a liquid such as a physiological saline can be injected from a syringe connected to the opening 2a.

Examples of the material that can be used to form the connector 2 include thermoplastic resins such as polycarbonate, polyamide, polysulfone, polyarylate, and methacrylate-styrene copolymer.

The balloon catheter 1 of the present embodiment is provided with a balloon 5, an outer shaft 3 that is coupled to a proximal end of the balloon 5 with a coupling part j2, an inner shaft 9 that is inserted into the outer shaft 3 and that is coupled to a distal end of the balloon 5 with a coupling part j1, and a distal end-side marker 7a and a proximal end-side marker 7b disposed on the inner shaft 9, wherein both ends of the distal end-side marker 7a are disposed astride a proximal end coupling position j1p of the coupling part j1 between the balloon 5 and the inner shaft 9 in the longitudinal direction of the inner shaft 9 (or the balloon catheter 1), and therefore, peeling can be prevented at the proximal end coupling position j1p between the balloon 5 and the inner shaft 9, and kinking can be prevented near the end section of the balloon 5 even when the balloon catheter 1 is curved.

Furthermore, because the both ends of the proximal end-side marker 7b are disposed astride the proximal end coupling position j2p of the coupling part j2 between the balloon 5 and the outer shaft 3 in the longitudinal direction of the inner shaft 9 (or the balloon catheter 1), peeling can be prevented at the proximal end coupling position j2p between the balloon 5 and the outer shaft 3, and kinking can be prevented near the end section of the balloon 5 even when the balloon catheter 1 is curved.

### (Second Embodiment)

Next, a second embodiment of the present invention will be described. The drawings used in the present embodiment are similarly exaggerated for ease of understanding, and the dimensions are different from the actual dimensions.

Hereinafter, the second embodiment of the present invention will be described, but because the overall description of the balloon catheter is the same as that of FIG. 1, the description thereof is omitted, and parts common to the first embodiment are given the same reference numerals and the description will be omitted.

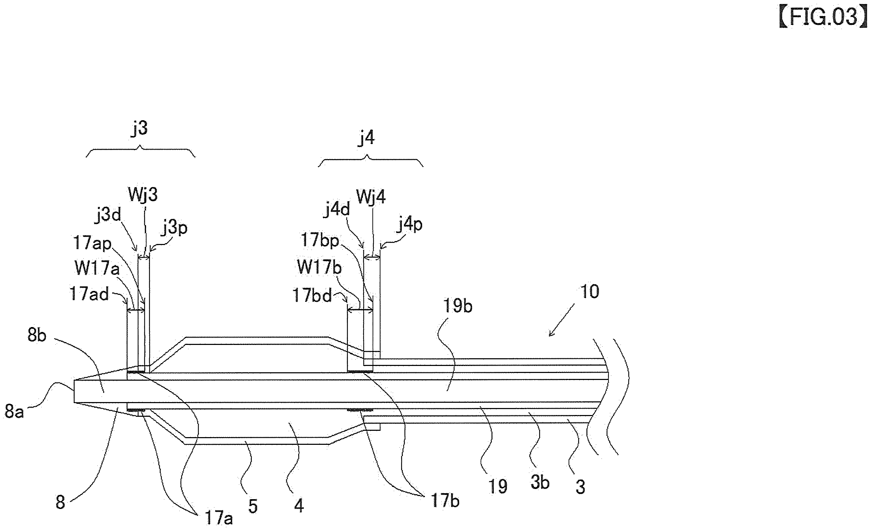

FIG. 3 is a longitudinal sectional view near the distal end section of a balloon catheter according to the second embodiment.

As shown in FIG. 3, the balloon catheter 10 of the present embodiment includes a long outer shaft 3, a balloon 5 provided on the distal end of the outer shaft 3, a distal tip 8 provided on the distal end of the balloon 5, an inner shaft 19 that is inserted into the outer shaft 3 and the balloon 5 and that is coupled to the distal end of the balloon 5, and a connector 2 provided on the proximal end of the outer shaft 3.

The inner shaft 19 of the present embodiment is made of the same material as the inner shaft 9 of the first embodiment, but it is formed slightly longer than the inner shaft 9 of the first embodiment because of a relationship with the position in which the distal end-side marker 17a described later is disposed.

That is to say, the inner shaft 19 is also a long and flexible tubular member, is inserted inside the outer shaft 3 and the balloon 5, is coupled to the distal end of the balloon 5, and the proximal end of the inner shaft 9 is coupled to the guide wire port 3a of the outer shaft 3, and is provided with a lumen 19b therein.

Furthermore, the inner shaft 19 of the present embodiment is provided with two radiopaque markers, namely a distal end-side marker 17a and a proximal end-side marker 17b which enable the position of the balloon 5 to be confirmed during a procedure.

Next, the relationship between the coupling position between the balloon 5 and the inner shaft 19, and the position of the distal end-side marker 17a will be described.

First, as shown in FIG. 3, the coupling position of the coupling part j3 between the balloon 5 and the inner shaft 19 (which corresponds to a "second coupling part" of the present invention) has a distal end coupling position j3d at the distal end, and a proximal end coupling position j3p at the position of a coupling width Wj3 on the proximal end side from the distal end coupling position j3d.

In contrast, the distal end position 17ad of the distal end-side marker 17a is positioned on the proximal end side of the distal end coupling position j3d of the coupling part j3 in the longitudinal direction of the inner shaft 19 (or the balloon catheter 10), and the proximal end position 17ap is at the position of a width W17a on the proximal end side from the distal end position 17ad, and is positioned on the distal end side of the proximal end coupling position j3p of the coupling part j3 in the longitudinal direction of the inner shaft 19 (or the balloon catheter 10).

That is to say, both ends of the distal end-side marker 17a are disposed astride the distal end coupling position j3d of the coupling part j3 between the balloon 5 and the inner shaft 19 in the longitudinal direction of the inner shaft 19 (or the balloon catheter 10).

Therefore, because the both ends of the distal end-side marker 17a are disposed astride the distal end coupling position j3d of the coupling part j3 between the balloon 5 and the inner shaft 19 in the longitudinal direction of the inner shaft 19 (or the balloon catheter 10), peeling can be prevented at the distal end coupling position j3d between the balloon 5 and the inner shaft 19, and kinking can be prevented near the end section of the balloon 5 even when the balloon catheter 10 is curved.

Next, the relationship between the coupling position between the balloon 5 and the outer shaft 3, and the position of the proximal end-side marker 17b will be described.

As shown in FIG. 3, the coupling position of the coupling part j4 between the balloon 5 and the outer shaft 3 (which corresponds to a "first coupling part" of the present invention) has a distal end coupling position j4d at the distal end, and a proximal end coupling position j4p at the position of a coupling width Wj4 on the proximal end side from the distal end coupling position j4d.

In contrast, the distal end position 17bd of the proximal end-side marker 17b is positioned on the distal end side of the distal end coupling position j4d of the coupling part j4 in the longitudinal direction of the inner shaft 19 (or the balloon catheter 10), and the proximal end position 17bp is at the position of a width W17b on the proximal end side from the distal end position 17bd, and is positioned on the distal end side of the proximal end coupling position j4p of the coupling part j4 in the longitudinal direction of the inner shaft 19 (or the balloon catheter 10).

That is to say, both ends of the proximal end-side marker 17b are disposed astride the distal end coupling position j4d of the coupling part j4 between the balloon 5 and the outer shaft 3 in the longitudinal direction of the inner shaft 19 (or the balloon catheter 10).

Therefore, because the both ends of the proximal end-side marker 17b are disposed astride the distal end coupling position j4d of the coupling part j4 between the balloon 5 and the outer shaft 3 in the longitudinal direction of the inner shaft 19 (or the balloon catheter 10), peeling can be prevented at the distal end coupling position j4d between the balloon 5 and the outer shaft 3, and kinking can be prevented near the end section of the balloon 5 even when the balloon catheter 10 is curved.

The same material as the distal end-side marker 7a and the proximal end-side marker 7b of the first embodiment can be used as the material forming the distal end-side marker 17a and the proximal end-side marker 17b.

The balloon catheter 10 of the present embodiment is provided with a balloon 5, an outer shaft 3 that is coupled to a proximal end of the balloon 5 with a coupling part j4, an inner shaft 19 that is inserted into the outer shaft 3 and that is coupled to a distal end of the balloon 5 with a coupling part j3, and a distal end-side marker 17a and a proximal end-side marker 17b disposed on the inner shaft 19, in which the both ends of the distal end-side marker 17a are disposed astride a distal end coupling position j3d of the coupling part j3 between the balloon 5 and the inner shaft 19 in the longitudinal direction of the inner shaft 19 (or the balloon catheter 10), and therefore, peeling can be prevented between at the distal end coupling position j3d between the balloon 5 and the inner shaft 19, and kinking can be prevented near the end section of the balloon 5 even when the balloon catheter 10 is curved.

Furthermore, because the both ends of the proximal end-side marker 17b are disposed astride the distal end coupling position j4d of the coupling part j4 between the balloon 5 and the outer shaft 3 in the longitudinal direction of the inner shaft 19 (or the balloon catheter 10), peeling can be prevented at the distal end coupling position j4d between the balloon 5 and the outer shaft 3, and kinking can be prevented near the end section of the balloon 5 even when the balloon catheter 10 is curved.

In the first embodiment, a case where both ends of the distal end-side marker 7a are disposed astride the proximal end coupling position j1p of the coupling part j1 between the balloon 5 and the inner shaft 9 (which corresponds to a "second coupling part" of the present invention) in the longitudinal direction of the inner shaft 9 (or the balloon catheter 1), and a case where both ends of the proximal end-side marker 7b are disposed astride the proximal end coupling position j2p of the coupling part j2 between the balloon 5 and the outer shaft 3 (which corresponds to a "first coupling part" of the present invention) in the longitudinal direction of the inner shaft 9 (or the balloon catheter 1) were described.

Furthermore, in the second embodiment, a case where both ends of the distal end-side marker 17a are disposed astride the distal end coupling position j3d of the coupling part j3 between the balloon 5 and the inner shaft 19 (which corresponds to a "second coupling part" of the present invention) in the longitudinal direction of the inner shaft 19 (or the balloon catheter 10), and a case where both ends of the proximal end-side marker 17b are disposed astride the distal end coupling position j4d of the coupling part j4 between the balloon 5 and the outer shaft 3 (which corresponds to a "first coupling part" of the present invention) in the longitudinal direction of the inner shaft 19 (or the balloon catheter 10) were described.

However, the present invention is not limited to the first embodiment and the second embodiment, and it is sufficient for both ends of the distal end-side marker to be disposed astride at least one of the distal end coupling position and the proximal end coupling position of the coupling part between the balloon and the inner shaft, and it is sufficient for both ends of the proximal end-side marker to be disposed astride at least one of the distal end coupling position and the proximal end coupling position of the coupling part between the balloon and the outer shaft. In this case, it is possible to prevent peeling of the end section of the coupling part in which the marker is disposed astride.

### (Third Embodiment)

Next, a third embodiment of the present invention will be described. The drawings used in the present embodiment are also exaggerated for ease of understanding, and the dimensions are different from the actual dimensions.

Hereinafter, the third embodiment of the present invention will be described, but because the overall description of the balloon catheter is the same as that in FIG. 1, the description thereof is omitted, and parts common to the first embodiment are given the same reference numerals and the description will be omitted.

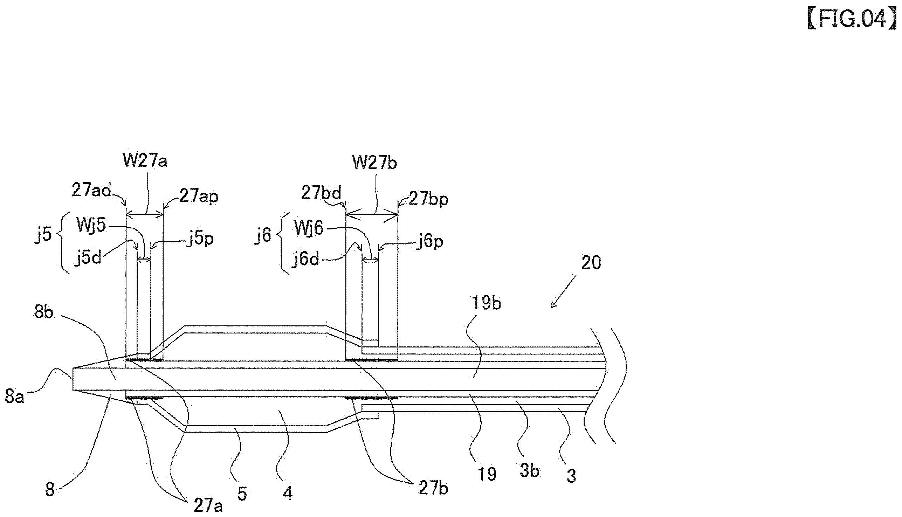

FIG. 4 is a longitudinal sectional view near the distal end section of a balloon catheter according to the third embodiment.

As shown in FIG. 4, the balloon catheter 20 of the present embodiment includes a long outer shaft 3, a balloon 5 provided on the distal end of the outer shaft 3, a distal tip 8 provided on the distal end of the balloon 5, an inner shaft 19 that is inserted into the outer shaft 3 and the balloon 5 and that is coupled to the distal end of the balloon 5, and a connector 2 provided on the proximal end of the outer shaft 3.

Furthermore, the inner shaft 19 of the present embodiment is provided with two radiopaque markers, namely a distal end-side marker 27a and a proximal end-side marker 27b which enable the position of the balloon 5 to be confirmed during a procedure.

Next, the relationship between the coupling position between the balloon 5 and the inner shaft 19, and the position of the distal end-side marker 27a in the present embodiment will be described.

First, as shown in FIG. 4, the coupling position of the coupling part j5 between the balloon 5 and the inner shaft 19 (which corresponds to a "second coupling part" of the present invention) has a distal end coupling position j5d at the distal end, and a proximal end coupling position j5p at the position of a coupling width Wj5 on the proximal end side from the distal end coupling position j5d.

In contrast, the distal end position 27ad of the distal end-side marker 27a is positioned on the distal end side of the distal end coupling position j5d of the coupling part j5 in the longitudinal direction of the inner shaft 19 (or the balloon catheter 20), and the proximal end position 27ap is at the position of a width W27a on the proximal end side from the distal end position 27ad, and is positioned on the proximal end side of the proximal end coupling position j5p of the coupling part j5 in the longitudinal direction of the inner shaft 19 (or the balloon catheter 20).

That is to say, both ends of the distal end-side marker 27a are disposed astride the distal end coupling position j5d and the proximal end coupling position j5p of the coupling part j5 between the balloon 5 and the inner shaft 19 in the longitudinal direction of the inner shaft 19 (or the balloon catheter 20).

Therefore, because the both ends of the distal end-side marker 27a are disposed astride the distal end coupling position j5d and the proximal end coupling position j5p of the coupling part j5 between the balloon 5 and the inner shaft 19 in the longitudinal direction of the inner shaft 19 (or the balloon catheter 20), peeling can be prevented at the distal end coupling position j5d and the proximal end coupling position j5p between the balloon 5 and the inner shaft 19, and kinking can be further prevented near the end section of the balloon 5 even when the balloon catheter 20 is curved.

Next, the relationship between the coupling position between the balloon 5 and the outer shaft 3, and the position of the proximal end-side marker 27b will be described.

As shown in FIG. 4, the coupling position of the coupling part j6 between the balloon 5 and the outer shaft 3 (which corresponds to the "first coupling part" of the present invention) has a distal end coupling position j6d at the distal end, and a proximal end coupling position j6p at the position of a coupling width Wj6 on the proximal end side from the distal end coupling position j6d.

In contrast, the distal end position 27bd of the proximal end-side marker 27b is positioned on the distal end side of the distal end coupling position j6d of the coupling part j6 in the longitudinal direction of the inner shaft 19 (or the balloon catheter 20), and the proximal end position 27bp is at the position of the width W27b on the proximal end side from the distal end position 27bd, and is positioned on the proximal end side of the proximal end coupling position j6p of the coupling part j6 in the longitudinal direction of the inner shaft 19 (or the balloon catheter 20).

That is to say, both ends of the proximal end-side marker 27b are disposed astride the distal end coupling position j6d and the proximal end coupling position j6p of the coupling part j6 between the balloon 5 and the outer shaft 3 in the longitudinal direction of the inner shaft 19 (or the balloon catheter 20).

Therefore, because the both ends of the proximal end-side marker 27b are disposed astride the distal end coupling position j6d and the proximal end coupling position j6p of the coupling part j6 between the balloon 5 and the outer shaft 3 in the longitudinal direction of the inner shaft 19 (or the balloon catheter 20), peeling can be prevented at the distal end coupling position j6d and the proximal end coupling position j6p of the coupling part j6 between the balloon 5 and the outer shaft 3, and kinking can be prevented near the end section of the balloon 5 even when the balloon catheter 20 is curved.

The same material as the distal end-side marker 7a and the proximal end-side marker 7b of the first embodiment can be used as the material forming the distal end-side marker 27a and the proximal end-side marker 27b.

The balloon catheter 20 of the present embodiment is provided with a balloon 5, an outer shaft 3 that is coupled to a proximal end of the balloon 5 with a coupling part j6, an inner shaft 19 that is inserted into the outer shaft 3 and that is coupled to a distal end of the balloon 5 with a coupling part j5, and a distal end-side marker 27a and a proximal end-side marker 27b disposed on the inner shaft 19, in which both ends of the distal end-side marker 27a are disposed astride a distal end coupling position j5d and a proximal end coupling position j5p of the coupling part j5 in the longitudinal direction of the inner shaft 19 (or the balloon catheter 20), and both ends of the proximal end-side marker 27b are disposed astride a distal end coupling position j6d and a proximal end coupling position j6p of the coupling part j6 in the longitudinal direction of the inner shaft 19 (or the balloon catheter 20), and therefore, peeling can be further prevented at the proximal end coupling position j5d and the proximal end coupling position j5p of the coupling part j5 between the balloon 5 and the inner shaft 19, and at the distal end coupling position j6d and the proximal end coupling position j6p of the coupling part j6 between the balloon 5 and the outer shaft 3, and kinking can be prevented near the end section of the balloon even when the balloon catheter 20 is curved.

In the present embodiment, a case where both ends of the distal end-side marker 27a are disposed astride the distal end coupling position j5d and the proximal end coupling position j5p of the coupling part j5 between the balloon 5 and the inner shaft 19 in the longitudinal direction of the inner shaft 19 (or the balloon catheter 20), and both ends of the proximal end-side marker 27b are disposed astride the distal end coupling position j6d and the proximal end coupling position j6p of the coupling part j6 between the balloon 5 and the outer shaft 3 in the longitudinal direction of the inner shaft 19 (or the balloon catheter 20) was described.

However, the present invention is not limited to this embodiment, and as long at least both ends of the distal end-side marker 27a or both ends of the proximal end-side marker 27b are disposed astride the distal end coupling position and the proximal end coupling position of the coupling part, peeling can be prevented at the distal end coupling position and the proximal end coupling position of the coupling part in which the marker are disposed astride, and kinking of the balloon can be prevented near the coupling part in which the marker is disposed astride.

### (Fourth Embodiment)

Next, a fourth embodiment of the present invention will be described. The drawings used in the present embodiment are also exaggerated for ease of understanding, and the dimensions are different from the actual dimensions.

Hereinafter, the fourth embodiment of the present invention will be described, but because the overall description of the balloon catheter is the same as that in FIG. 1, the description thereof is omitted, and parts common to the first embodiment are given the same reference numerals and the description will be omitted.

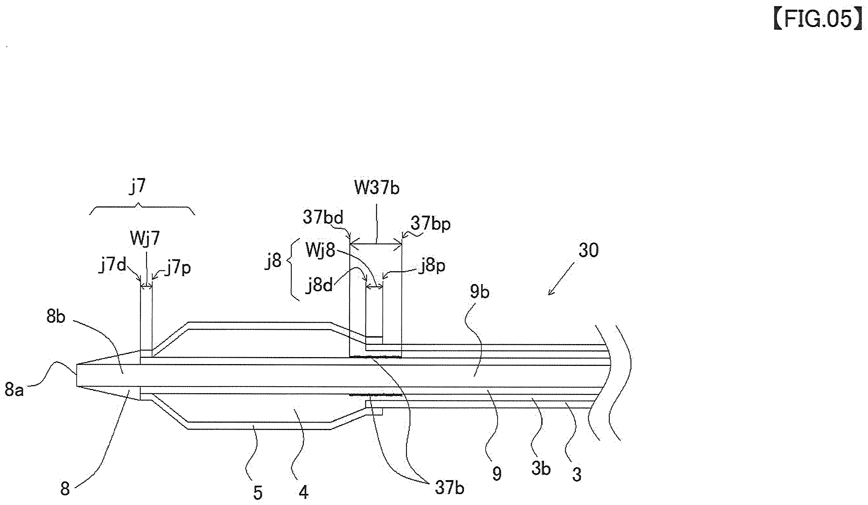

FIG. 5 is a longitudinal sectional view near the distal end section of a balloon catheter according to a fourth embodiment.

As shown in FIG. 5, the balloon catheter 30 of the present embodiment includes a long outer shaft 3, a balloon 5 provided on the distal end of the outer shaft 3, a distal tip 8 provided on the distal end of the balloon 5, an inner shaft 9 that is inserted into the outer shaft 3 and the balloon 5 and that is coupled to the distal end of the balloon 5, and a connector 2 provided on the proximal end of the outer shaft 3.

Furthermore, the inner shaft 9 of the present embodiment is provided with a radiopaque proximal end-side marker 37b which enables the position of the balloon 5 to be confirmed during a procedure.

Furthermore, the inner shaft 9 of the present embodiment is not provided with a radiopaque marker on the distal end side of the balloon 5. However, as shown in FIG. 5, the coupling position of the coupling part j7 between the balloon 5 and the inner shaft 9 has a distal end coupling position j7d at the distal end, and a proximal end coupling position j7p at the position of a coupling width Wj7 on the proximal end side from the distal end coupling position j7d.

Here, the relationship between the coupling position between the balloon 5 and the outer shaft 3, and the position of the proximal end-side marker 37b will be described.

As shown in FIG. 5, the coupling position of the coupling part j8 between the balloon 5 and the outer shaft 3 (which corresponds to a "first coupling part" of the present invention) has a distal end coupling position j8d at the distal end, and a proximal end coupling position j8p at the position of a coupling width Wj8 on the proximal end side from the distal end coupling position j8d.

In contrast, the distal end position 37bd of the proximal end-side marker 37b is positioned on the distal end side of the distal end coupling position j8d of the coupling part j8 in the longitudinal direction of the inner shaft 9 (or the balloon catheter 30), and the proximal end position 37bp is at the position of a width W37b on the proximal end side from the distal end position 37bd, and is positioned on the proximal end side of the proximal end coupling position j8p of the coupling part J8 in the longitudinal direction of the inner shaft 9 (or the balloon catheter 30).

That is to say, both ends of the proximal end-side marker 37b are disposed astride the distal end coupling position j8d and the proximal end coupling position j8p of the coupling part j8 between the balloon 5 and the outer shaft 3 in the longitudinal direction of the inner shaft 9 (or the balloon catheter 30).

Therefore, because the both ends of the proximal end-side marker 37b are disposed astride the distal end coupling position j8d and the proximal end coupling position j8p of the coupling part J8 between the balloon 5 and the outer shaft 3 in the longitudinal direction of the inner shaft 9 (or the balloon catheter 30), peeling can be prevented at the distal end coupling position j8d and the proximal end coupling position j8p of the coupling part j8 between the balloon 5 and the outer shaft 3, and kinking can be prevented near the end section of the balloon 5 even when the balloon catheter 30 is curved.

The same material as the distal end-side marker 7a and the proximal end-side marker 7b of the first embodiment can be used as the material forming the proximal end-side marker 37b.

The balloon catheter 30 of the present embodiment is provided with a balloon 5, an outer shaft 3 that is coupled to a proximal end of the balloon 5 with a coupling part J8, an inner shaft 9 that is inserted into the outer shaft 3 and that is coupled to a distal end of the balloon 5 with a coupling part j7, and a proximal end-side marker 37b disposed on the inner shaft 9, in which a marker is not disposed on the distal end-side coupling part j7 and is disposed on the proximal end-side coupling part J8, and therefore, the distal end section of the balloon catheter 30 can be made even thinner and softer.

Furthermore, because the both ends of the distal end-side marker 37b are disposed astride the distal end coupling position j8d and the proximal end coupling position j8p of the proximal end-side coupling part J8 in the longitudinal direction of the inner shaft 9 (or the balloon catheter 30), peeling can be prevented at the distal end coupling position j8d and the proximal end coupling position j8p between the balloon 5 and the outer shaft 3, and kinking can be prevented near the end section of the balloon 5 even when the balloon catheter 30 is curved.

### (Fifth Embodiment)

Next, a fifth embodiment of the present invention will be described. The drawings used in the present embodiment are also exaggerated for ease of understanding, and the dimensions are different from the actual dimensions.

Hereinafter, the fifth embodiment of the present invention will be described, but because the overall description of the balloon catheter is the same as that in FIG. 1, the description thereof is omitted, and parts common to the first embodiment are given the same reference numerals and the description will be omitted.

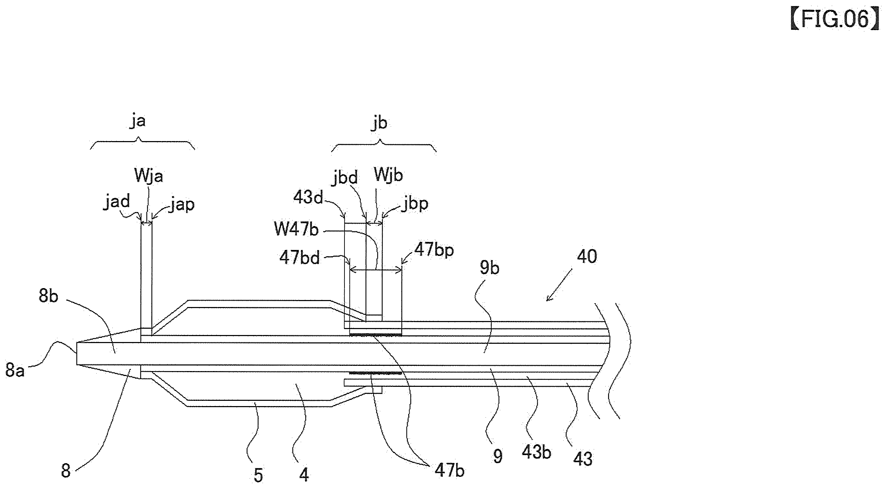

FIG. 6 is a longitudinal sectional view near the distal end section of a balloon catheter according to the fifth embodiment.

As shown in FIG. 6, the balloon catheter 40 of the present embodiment includes a long outer shaft 43, a balloon 5 provided on the distal end section of the outer shaft 43, a distal tip 8 provided on the distal end of the balloon 5, an inner shaft 9 that is inserted into the outer shaft 43 and the balloon 5 and that is coupled to the distal end of the balloon 5, and a connector 2 provided on the proximal end of the outer shaft 3.

The outer shaft 43 is a long and flexible tubular member, and as shown in FIG. 6, the distal end 43d extends beyond the distal end of the coupling part jb between the proximal end of the balloon 5 and the outer shaft 43 on the distal end side, and the proximal end is coupled to the distal end of the connector 2.

The outer shaft 43 has an inner shaft 9 inserted therein, and has a lumen 43b between the outer shaft and the inner shaft 9 that communicates the internal space 4 of the balloon 5 with a lumen inside the connector 2 (not illustrated).

Furthermore, along the longitudinal direction, the outer shaft 43 is provided with, in a similar fashion to the outer shaft 3, a guide wire port 43a (not illustrated) which serves as an opening for inserting a guide wire, and the guide wire port 43a is coupled to the proximal end of the inner shaft 9.

The same material as the material forming the outer shaft 3 can be used to form the outer shaft 43.

The inner shaft 9 of the present embodiment is provided with a radiopaque proximal end-side marker 47b which enables the position of the balloon 5 to be confirmed during a procedure.

Furthermore, the inner shaft 9 of the present embodiment is not provided with a radiopaque marker on the distal end side of the balloon 5. However, as shown in FIG. 6, the coupling position of the coupling part ja between the balloon 5 and the inner shaft 9 has a distal end coupling position jad at the distal end, and a proximal end coupling position jap at the position of a coupling width Wja on the proximal end side from the distal end coupling position jad.

Here, the relationship between the coupling position between the balloon 5 and the outer shaft 43, and the position of the proximal end-side marker 47b will be described.

As shown in FIG. 6, the coupling position of the coupling part jb between the balloon 5 and the outer shaft 43 (which corresponds to a "first coupling part" of the present invention) has a distal end coupling position jbd at the distal end, and a proximal end coupling position jbp at the position of a coupling width Wjb on the proximal end side from the distal end coupling position jbd.

In contrast, the distal end position 47bd of the proximal end-side marker 47b is positioned on the distal end side of the distal end coupling position jbd of the coupling part jb in the longitudinal direction of the inner shaft 9 (or the balloon catheter 40), and the proximal end position 47bp is at the position of a width W47b on the proximal end side from the distal end position 47bd, and is positioned on the proximal end side of the proximal end coupling position jbp of the coupling part jb in the longitudinal direction of the inner shaft 9 (or the balloon catheter 40).

That is to say, both ends of the proximal end-side marker 47b are disposed astride the distal end coupling position jbd and the proximal end coupling position jbp of the coupling part jb between the balloon 5 and the outer shaft 43 in the longitudinal direction of the inner shaft 9 (or the balloon catheter 40).

Further, as mentioned above, the distal end 43d of the outer shaft 43 extends beyond the distal end jbd of the coupling part jb on the distal end side.

Therefore, because the both ends of the proximal end-side marker 47b are disposed astride the distal end coupling position jbd and the proximal end coupling position jbp of the coupling part jb between the balloon 5 and the outer shaft 43 in the longitudinal direction of the inner shaft 9 (or the balloon catheter 40), and the distal end 43d of the outer shaft 43 extends beyond the distal end coupling position jbd of the coupling part jb on the distal end side, the change in rigidity near the distal end section of the balloon 5 is made more gradual in the longitudinal direction, and therefore, peeling can be further prevented at the distal end coupling position jbd and the proximal end coupling position jbp between the balloon 5 and the outer shaft 43, and kinking can be further prevented near the end section of the balloon 5 even when the balloon catheter 40 is curved.

The same material as the distal end-side marker 7a and the proximal end-side marker 7b of the first embodiment can be used as the material forming the proximal end-side marker 47b.

The balloon catheter 40 of the present embodiment is provided with a balloon 5, an outer shaft 43 that is coupled to a proximal end of the balloon 5 with a coupling part jb, an inner shaft 9 that is inserted into the outer shaft 43 and that is coupled to a distal end of the balloon 5 with a coupling part ja, and a proximal end-side marker 37b disposed on the inner shaft 9, in which a marker is not disposed on the coupling part ja and is disposed on the coupling part jb, and therefore, the distal end section of the balloon catheter 40 can be made even thinner and softer.

Furthermore, because the both ends of the proximal end-side marker 47b are disposed astride the distal end coupling position jbd and the proximal end coupling position jbp of the coupling part jb between the balloon 5 and the outer shaft 43 in the longitudinal direction of the inner shaft 9 (or the balloon catheter 40), and the distal end 43d of the outer shaft 43 extends beyond the distal end jbd of the coupling part jb on the distal end side, the change in rigidity near the distal end section of the balloon 5 is made more gradual in the longitudinal direction, and therefore, peeling can be further prevented at the distal end coupling position jbd and the proximal end coupling position jbp between the balloon 5 and the outer shaft 43, and kinking can be further prevented near the end section of the balloon 5 even when the balloon catheter 40 is curved.

### (Sixth Embodiment)

Next, a sixth embodiment of the present invention will be described. The drawings used in the present embodiment are also exaggerated for ease of understanding, and the dimensions are different from the actual dimensions.

Hereinafter, the sixth embodiment of the present invention will be described, but because the overall description of the balloon catheter is the same as that in FIG. 1, the description thereof is omitted, and parts common to the first embodiment are given the same reference numerals and the description will be omitted.

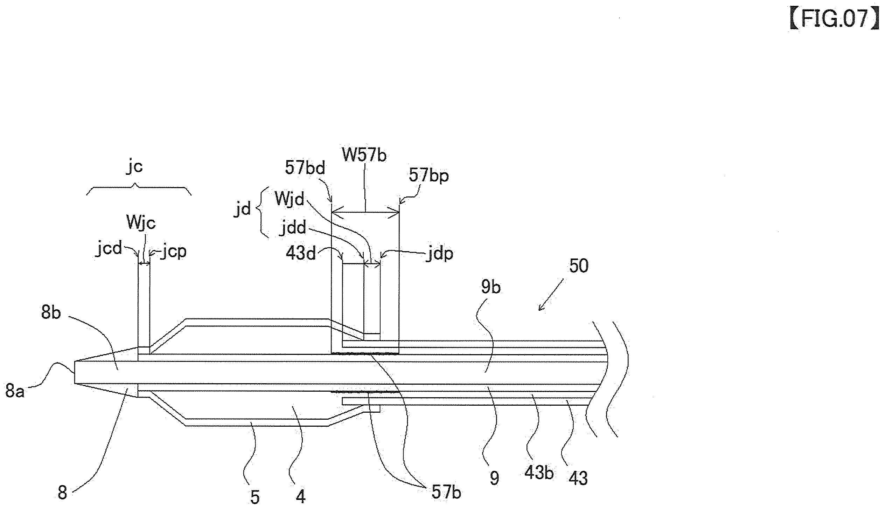

FIG. 7 is a longitudinal sectional view near the distal end section of a balloon catheter according to the sixth embodiment.

As shown in FIG. 7, the balloon catheter 50 of the present embodiment includes a long outer shaft 43, a balloon 5 provided on the distal end of the outer shaft 43, a distal tip 8 provided on the distal end of the balloon 5, an inner shaft 9 that is inserted into the outer shaft 43 and the balloon 5 and that is coupled to the distal end of the balloon 5, and a connector 2 provided on the proximal end of the outer shaft 3.

The inner shaft 9 of the present embodiment is provided with a radiopaque proximal end-side marker 57b which enables the position of the balloon 5 to be confirmed during a procedure.

Furthermore, the inner shaft 9 of the present embodiment is not provided with a radiopaque marker on the distal end side of the balloon 5. However, as shown in FIG. 7, the coupling position of the coupling part jc between the balloon 5 and the inner shaft 9 has a distal end coupling position jcd at the distal end, and a proximal end coupling position jcp at the position of a coupling width Wjc on the proximal end side from the distal end coupling position jcd.

Here, the relationship between the coupling position between the balloon 5 and the outer shaft 43, and the position of the proximal end-side marker 57b will be described.

As shown in FIG. 7, the coupling position of the coupling part jd between the balloon 5 and the outer shaft 43 (which corresponds to a "first coupling part" of the present invention) has a distal end coupling position jdd at the distal end, and a proximal end coupling position jdp at the position of a coupling width Wjd on the proximal end side from the distal end coupling position jdd.

In contrast, the distal end position 57bd of the proximal end-side marker 57b is positioned on the distal end side of the distal end coupling position jdd of the coupling part jd in the longitudinal direction of the inner shaft 9 (or the balloon catheter 50), and is positioned on the distal end side of the distal end 43d of the outer shaft 43 in the longitudinal direction of the inner shaft 9 (or the balloon catheter 50).

Furthermore, the proximal end position 57bp is at the position of a width W57b on the proximal end side from the distal end position 57bd, and is positioned on the proximal end side of the proximal end coupling position jdp of the coupling part jd in the longitudinal direction of the inner shaft 9 (or the balloon catheter 50).

That is to say, both ends of the proximal end-side marker 57b are disposed astride the distal end coupling position jdd and the proximal end coupling position jdp of the coupling part jd between the balloon 5 and the outer shaft 43 in the longitudinal direction of the inner shaft 9 (or the balloon catheter 50).

Further, as mentioned above, the distal end position 57bd of the proximal end-side marker 57b extends beyond the distal end 43d of the outer shaft 43 on the distal end side in the longitudinal direction of the inner shaft 9 (or the balloon catheter 50).

Therefore, because the both ends of the proximal end-side marker 57b are disposed astride the distal end coupling position jdd and the proximal end coupling position jdp of the coupling part jd between the balloon 5 and the outer shaft 43 in the longitudinal direction of the inner shaft 9 (or the balloon catheter 50), the distal end of the outer shaft 43 extends beyond the distal end jdd of the coupling part jd on the distal end side, and the distal end position 57bd of the proximal end-side marker 57b extends beyond the distal end 43d of the outer shaft 43 on the distal end side, peeling can be further prevented at the distal end coupling position jdd and the proximal end coupling position jdp between the balloon 5 and the outer shaft 43, and kinking can be prevented near the end section of the balloon 5 even when the balloon catheter 50 is curved, and further, the balloon can be deflated more safely by preventing the necking phenomenon from occurring at the proximal end section of the balloon 5.

The same material as the distal end-side marker 7a and the proximal end-side marker 7b of the first embodiment can be used as the material forming the proximal end-side marker 57b.

The balloon catheter 50 of the present embodiment is provided with a balloon 5, an outer shaft 43 that is coupled to a proximal end of the balloon 5 with a coupling part jd, an inner shaft 9 that is inserted into the outer shaft 43 and that is coupled to a distal end of the balloon 5 with a coupling part jc, and a proximal end-side marker 57b disposed on the inner shaft 9, in which a marker is not disposed on the coupling part jc but is disposed on the side coupling part jd, and therefore, the distal end section of the balloon catheter 50 can be made even thinner and softer.

Furthermore, because the both ends of the proximal end-side marker 57b are disposed astride the distal end coupling position jdd and the proximal end coupling position jdp of the coupling part jd between the balloon 5 and the outer shaft 43 in the longitudinal direction of the inner shaft 9 (or the balloon catheter 50), the distal end of the outer shaft 43 extends beyond the distal end coupling position jdd of the coupling part jd on the distal end side, and further, the distal end position 57bd of the proximal end-side marker 57b extends beyond the distal end 43d of the outer shaft 43 on the distal end side, peeling can be further prevented at the distal end coupling position jdd and the proximal end coupling position jdp between the balloon 5 and the outer shaft 43, and kinking can be prevented near the end section of the balloon 5 even when the balloon catheter 50 is curved, and further, the balloon can be deflated more safely by preventing the necking phenomenon from occurring at the proximal end section of the balloon 5.

The balloon catheter according to various embodiments of the present invention has been described above, however, the present invention is not limited to the above embodiments, and various modifications can be made without departing from the scope of the invention.

For example, the fourth embodiment to the sixth embodiment were described with no radiopaque marker being provided at a position on the inner shaft 9 corresponding to the distal end side of the balloon 5, however, if the distal end section of the balloon catheter does not need to be made thinner, or the distal end section of the catheter does not need to be made soft, a radiopaque marker can be provided at a position corresponding to the distal end side of the balloon 5 in the same manner as the balloon catheter 1 of the first embodiment and the balloon catheter 10 of the second embodiment.

### DESCRIPTION OF REFERENCE NUMERALS

- 1, 10, 20, 30, 40, 50: Balloon catheter
- 2: Connector
- 3, 43: Outer shaft
- 4: Internal space
- 5: Balloon
- 8: Distal tip
- 9, 19: Inner shaft
- 7a, 17a, 27a: Distal end-side marker
- 7b, 17b, 27b, 37b, 47b, 57b: Proximal end-side marker
- j1, j2, j3, j4, j5, j6, j7, j8, ja, jb, jc, jd: Coupling part

## Claims

1. A balloon catheter comprising:
a balloon;
an outer shaft that is coupled to a proximal end of the balloon with a first coupling part;
an inner shaft that is inserted into the outer shaft and that is coupled to a distal end of the balloon with a second coupling part; and
a marker disposed on the inner shaft, wherein
both ends of the marker are disposed astride at least one of, a distal end and a proximal end of the first coupling part, and a distal end and a proximal end of the second coupling part in a longitudinal direction of the inner shaft.

2. The balloon catheter according to claim 1, wherein the both ends of the marker are disposed astride the distal end and the proximal end of the first coupling part, or the distal end and the proximal end of the second coupling part in the longitudinal direction of the inner shaft.

3. The balloon catheter according to claim 1 or 2, wherein the distal end of the outer shaft extends beyond the distal end of the first coupling part on a distal end side.

4. The balloon catheter according to claim 3, wherein a distal end of the marker extends beyond a distal end of the outer shaft on the distal end side.

5. The balloon catheter according to any one of claims 1 to 4, wherein the marker is not disposed on the second coupling part, and is disposed on the first coupling part.
